# EUROPEAN PATENT APPLICATION

(11) **EP 4 166 500 A1**
(43) Date of publication of application: **19.04.2023**
(21) Application number: 21822711.4
(22) Date of filing: 09.06.2021
(51) Int. Cl.: C01B 33/158, C09C 1/28, C09C 1/62, A61Q 1/00, A61Q 19/00, A61K 8/25

(54) **SPHERICAL SILICA PARTICLE, AND METHOD FOR PRODUCING SAME**

(30) Priority: 12.06.2020 JP 2020102443
(71) Applicant: Tayca Corporation, Osaka-shi, Osaka 551-0022 (JP)
(72) Inventor: KANDA Naoki, Osaka-shi, Osaka 551-0022 (JP); OOSAKI Daisuke, Osaka-shi, Osaka 551-0022 (JP); TANAKA Toru, Osaka-shi, Osaka 551-0022 (JP)
(74) Representative: Reitstötter Kinzebach
(86) International application number: PCT/JP2021/021986
(87) International publication number: WO 2021/251440

(57) **Abstract**

Provided are porous spherical silica particles whose oil absorption is suppressed while the porous spherical silica particles have a large specific surface area; and a method for manufacturing such spherical silica particles.

According to the present invention, provided are spherical silica particles whose specific surface area obtained by employing a BET method is 300 m²/g or more, total pore volume is 0.3 ml/g or less, and oil absorption is 50 ml/100g or less, the spherical silica particles obtained by subjecting silica gel particles obtained by employing a sol-gel method, for example, in which an alkali silicate is emulsified and coagulated, to only drying at a low temperature without subjecting the silica gel particles to calcination at a high temperature; and a method for manufacturing such spherical silica particles.

## Description

### TECHNICAL FIELD

The present invention relates to porous spherical silica particles whose oil absorption is suppressed while the spherical silica particles have a large specific surface area and a method for manufacturing such spherical silica particles.

### BACKGROUND ART

Conventionally, it has been known that in a multitude of cosmetics, for the purpose of enhancing sebum adsorption ability and texture, porous spherical silica particles are blended. For example, Japanese Patent Application Laid-Open Publication No. 2018-177620 (Patent Literature 1) discloses a silica aerogel powder which has acquired high oil absorption characteristics by enhancing porosity of silica aerogel by a pore control technology and which has excellent rolling properties to skin by subjecting the silica aerogel to spheroidization while strength of the silica aerogel made porous is enhanced by a shape control technology and allows smooth texture to be obtained.

However, since the silica aerogel powder described in Patent Literature 1 has high oil absorption, when the silica aerogel powder is blended in a cosmetic, the silica aerogel powder adsorbs an oil phase component in the cosmetic, thereby leading to a problem in that it is difficult to prescribe the cosmetic so as to obtain predetermined components. Therefore, in order to solve the above-mentioned problem, for example, a countermeasure such as lowering oil absorption of silica particles by pore control or enhancing dispersibility into an oil phase by imparting hydrophobicity to the silica particles by surface treatment has been taken.

As a method for lowering the oil absorption of the silica particles, there has been known a method in which the oil absorption is lowered by controlling a calcination temperature, for example, in drying and calcination processes of the particles and thereby decreasing a pore volume. However, when the oil absorption of the silica particles is lowered by employing this method, since a surface area of the silica particles is reduced due to mutual sintering of the particles or a decrease in the number of pores and conversely, a proportion of an area contacting skin increases, slipping of the silica particles is worsened, thereby leading to a problem in that smooth texture cannot be obtained. In addition, in a case where calcination is conducted, since dehydration condensation inside a particle structure progresses, the silica particles are felt to be further hard, thereby leading to also a problem in that texture is worsened.

As another method for lowering the oil absorption of the silica particles, there has been known a method in which the silica particles are hydrophobized by, for example, a reactive silane coupling agent such as silicone or alkylsilane for surface treatment. However, when the silica particles are hydrophobized, although blending thereof into the oil phase is facilitated by enhancement in dispersibility into an oil agent, since a hydroxyl group on a surface of the silica particles cannot be completely coated, phase inversion to a water phase is caused over time, thereby leading to a problem in that stability of a cosmetic formulation is worsened.

In addition, although it is considered that silica gel particles are forcibly blended into the water phase by adding a surfactant or conducting forceful stirring, since it is difficult to completely suppress clumping of the particles in the water phase, tackiness attributable to the addition of the surfactant and squeakiness or the like due to particle clumping are caused, thereby leading to a problem in that texture is worsened.

### CITATION LIST

### PATENT LITERATURE

Patent Literature 1: Japanese Patent Application Laid-Open Publication No. 2018-177620

### SUMMARY OF THE INVENTION

### TECHNICAL PROBLEM

Therefore, objects of the present invention are to provide spherical silica particles whose oil absorption is suppressed while the spherical silica particles have a large specific surface area and which are excellent in adherability to skin and texture and a method for manufacturing such spherical silica particles.

### SOLUTION TO PROBLEM

The present inventors have devoted themselves to earnest research as to a method for manufacturing silica particles whose oil absorption is suppressed while the silica particles have a large specific surface area. As a result, the present inventors have found out that by forming a W/O emulsion; thereafter, performing gelation and separation by heating; washing a gelled body included in a separated W phase; and performing only drying at a low temperature without performing calcination at a high temperature, porous spherical silica particles whose oil absorption is suppressed while the spherical silica particles have a large specific surface area can be obtained, thereby reaching the completion of the present invention.

In order for silica particles compounded in cosmetics or the like to exhibit use feeling excellent in adherability to skin and texture, it is desired that a specific surface area obtained by employing a BET method is 300 m²/g or more, a total pore volume is 0.3 ml/g or less, and oil absorption is suppressed to 50 ml/100g or less.

It is because that the specific surface area obtained by employing the BET method is made to be 300 m²/g or more, thereby allowing a proportion of an area of the silica particles contacting skin to be sufficiently decreased and whereby upon applying the silica particles to skin, hardness of the silica particles is hardly felt.

In addition, it is because that the specific surface area obtained by employing the BET method is made to be 300 m²/g or more and the total pore volume is made to be 0.3 ml/g or less (in other words, while the specific surface area is made large, the pore volume is made small), whereby the oil absorption can be suppressed to a low level such as 50 ml/100g or less and adverse influence (aggregation of the silica particles and dry texture of skin) due to excessive absorption of an oil content is hardly caused.

With respect to these targets, according to the present invention, provided are spherical silica particles whose at least specific surface area obtained by employing the BET method is 300 m²/g or more, whose at least total pore volume is 0.3 ml/g or less, and whose at least oil absorption is 50 ml/100g or less. In addition, the above-described spherical silica particles have a characteristic in that in a manufacturing step, the spherical silica particles are not subjected to calcination processing at a temperature of 1000°C or more.

In the silica gel obtained by forming the W/O emulsion; and thereafter, performing the gelation and separation of-the W phase in which the silica gel is included by the heating, moisture is retained. This moisture retained in the silica gel is divided into adhesion water and structural water, and although ordinarily, the adhesion water can be easily removed by heating at a temperature of approximately 100°C, it is difficult to remove the structural water by heating even at a temperature of 400°C or more. Therefore, as to the spherical silica particles of the present invention whose manufacturing method omits the calcination processing, a decrease in the specific surface area and a decrease in the pore volume, which are caused by proceeding of densification or the like, are suppressed, and the spherical silica particles have a characteristic in that a content percentage of structural water is 1.6% or more and more preferably, the content percentage thereof is 2.0% or more. By having the above-mentioned characteristic, the spherical silica particles of the present invention have favorable moist texture.

The silica particles of the present invention are spherical, and the oil absorption thereof is suppressed while the silica particles are porous and have the large specific surface area. Furthermore, since particles of the silica particles of the present invention are hardly broken even when used in cosmetic applications, the silica particles can exhibit use feeling excellent in the adherability to skin and texture.

In addition, in the spherical silica particles of the present invention, one kind or more of a metal oxide or metal oxides selected from the group consisting of a titanium oxide, a zinc oxide, an iron oxide, and an aluminum oxide may be compounded, and in such a case, characteristics derived from the metal oxide or metal oxides (for example, an ultraviolet ray shielding effect, a coloring effect, and the like) can be imparted to the silica particles. At this time, preferably, a content rate of the metal oxide or metal oxides to the whole of the silica particles is 0.5 wt.% to 30 wt.% and more preferably, the content rate thereof is 5 wt.% to 20 wt.%.

In addition, the spherical silica particles of the present invention may be subjected to surface treatment by a reactive silane coupling agent such as silicone and alkylsilane and may be thereby hydrophobized, and in such a case, dispersibility thereof to an oil agent is enhanced.

Furthermore, according to the present invention, provided is a method for manufacturing spherical silica particles which includes:
(1) a step of forming a W/O type emulsion in which an alkali silicate aqueous solution is a dispersed phase and a liquid which cannot mix with the alkali silicate aqueous solution is a continuous phase;
(2) a step of generating a spherical silica gel by mixing the W/O type emulsion with a mineral acid aqueous solution;
(3) a step of performing separation into two layers which are an O phase and a W phase and removing the O phase by heating a reaction liquid including the generated spherical silica gel;
(4) a step of washing the W phase including the spherical silica gel from which the O phase has been removed; and
(5) a step of drying the washed spherical silica gel.

In addition, in order to obtain spherical silica particles having a desired average primary particle diameter, the dried silica particles may be crushed or be classified.

According to the manufacturing method of the present invention, as described above, the porous spherical silica particles whose oil absorption is suppressed to 50 ml/100g or less while the porous spherical silica particles have the large specific surface area of 300 m²/g or more obtained by employing the BET method can be obtained. In particular, it is effective not to perform the calcination treatment after the step (5).

### <Preparation of Emulsion>

An alkali silicate aqueous solution, a liquid, such as a non-polar solvent, which does not mix with the alkali silicate aqueous solution, and an emulsifying agent are blended; and the blended liquid is emulsified by using an emulsifying apparatus such as a stirring type emulsifying apparatus, a high pressure type emulsifying apparatus, an ultrasonic type emulsifying apparatus, and a membrane emulsification type emulsifying apparatus. Thereby forming a W/O type emulsion in which the liquid such as the non-polar solvent is a continuous phase and the alkali silicate aqueous solution is a dispersed phase which is dispersed in a fine-granular state.

A particle diameter of the dispersed phase can be controlled by changing output of the emulsifying apparatus upon emulsifying. For example, when the emulsifying apparatus is a stirring type emulsifying apparatus having rotary stirring blades, the more the number of revolutions of the stirring blades is increased, the smaller the particle diameter becomes, and the more the number of revolutions is decreased, the larger the particle diameter becomes. In addition, the particle diameter of the dispersed phase can be controlled also by changing a concentration of the alkali silicate aqueous solution. For example, the concentration of the alkali silicate aqueous solution is reduced, thereby allowing the particle diameter to be made further fine, and conversely, the concentration is increased, thereby increasing viscosity and allowing the particle diameter to be made large.

As the alkali silicate used in the present invention, a sodium silicate, a potassium silicate, a lithium silicate, and the like can be cited, and in particular, the sodium silicate is suitably used. In addition, the alkali silicate aqueous solution can be prepared by dissolving natural silica or synthetic silica in an alkali aqueous solution such as a sodium hydroxide aqueous solution.

In addition, one kind or more of a metal oxide or metal oxides selected from the group consisting of a titanium oxide, a zinc oxide, an iron oxide, and an aluminum oxide or a precursor compound or precursor compounds of one kind or more of the metal oxide or metal oxides selected from the group consisting of the titanium oxide, the zinc oxide, the iron oxide, and the aluminum oxide may be added to the alkali silicate aqueous solution. Note that as the precursor compound or precursor compounds thereof, for example, a hydroxide, salt, an alkoxide, and the like are cited. It is only required for an added amount (expressed in terms of an oxide) of the metal oxide or metal oxides or the precursor compound or precursor compounds to calculate a content rate to the whole particles and to adjust the added amount based on a value of the content rate.

The liquid, used in the present invention, which does not mix with the alkali silicate aqueous solution, is not particularly limited as long as the liquid does not react with the later-described mineral acid aqueous solution, and for example, aromatic hydrocarbons such as toluene, xylene, ethylbenzene, and tetralin, aliphatic hydrocarbons such as n-octane, gasoline, kerosene, and isoparaffinic hydrocarbon oil, alicyclic hydrocarbons such as cyclononane and cyclodecane, and the like can be cited, and from the point of view of obtainment of homogeneous and stable dispersibility, it is preferable that each of the aromatic hydrocarbons such as xylene is used.

The emulsifying agent used in the present invention is not particularly limited if the emulsifying agent has a function to stabilize the W/O type emulsion, and a strongly lipophilic surfactant such as fatty acid multivalent metal salt and poorly water-soluble cellulose ether can be used.

As the surfactant, it is preferable that a non-ionic surfactant is used, and as specific examples, sorbitan fatty acid ester such as sorbitan monolaurate, sorbitan monopalmitate, sorbitan monostearate, sorbitan distearate, sorbitan tristearate, sorbitan monooleate, sorbitan sesquioleate, and sorbitan trioleate; polyoxyethylene sorbitan fatty acid ester such as polyoxyethylene sorbitan monolaurate, polyoxymethylene sorbitan monopalmitate, polyoxyethylene sorbitan monostearate, and polyoxyethylene sorbitan monooleate; polyoxyethylene fatty acid ester such as polyoxyethylene monolaurate, polyoxyethylene monopalmitate, polyoxyethylene monostearate, and polyoxyethylene monooleate; glycerin fatty acid ester such as stearic acid monoglyceride and oleic acid monoglyceride; and the like can be cited.

### <Generation of Silica Gel>

The W/O type emulsion including as the dispersed phase the alkali silicate aqueous solution prepared by the above-described emulsifying step is blended with the mineral acid aqueous solution, and mineral acid and alkali silicate cause neutralization reaction, thereby generating a porous spherical silica gel. Although a method of blending the W/O type emulsion and the mineral acid aqueous solution is not particularly limited, since upon adding the mineral acid aqueous solution to the W/O type emulsion including the alkali silicate aqueous solution, an excessive reduction in a concentration of the mineral acid upon the neutralization reaction may be caused, it is preferable that the W/O type emulsion including the alkali silicate aqueous solution is added to the mineral acid aqueous solution while the mineral acid aqueous solution is being stirred.

As the mineral acid used in the present invention, sulfuric acid, nitric acid, hydrochloric acid, and the like can be cited, and in general, it is preferable that the sulfuric acid whose dehydration action is strong and which is advantageous also in terms of costs is used.

Preferably, the W/O type emulsion including the alkali silicate aqueous solution is blended with the sulfuric acid aqueous solution having a concentration of 15 wt.% or more, more preferably, the W/O type emulsion including the alkali silicate aqueous solution is blended with the sulfuric acid aqueous solution having a concentration of 30 wt.% or more, and it is preferable that an upper limit of the concentration of the sulfuric acid aqueous solution is 50 wt.% or less. If the concentration of the mineral acid aqueous solution is less than 15 wt.%, a coarse particle-state silica gel is easily generated, and if the concentration of the sulfuric acid aqueous solution is increased to 50 wt.% or more, it is likely that sphericity of generated silica gel particles is reduced. Note that it is preferable that the mineral acid aqueous solution, whose amount generates free mineral acid which does not contribute to generation of salt after the neutralization reaction, is blended with the W/O type emulsion including the alkali silicate aqueous solution.

The neutralization reaction of the W/O type emulsion including the alkali silicate aqueous solution and the mineral acid aqueous solution is finished, ordinarily, after five minutes to 120 minutes, though it depends on blend conditions and the like, and finishing of the neutralization reaction can be confirmed by starting of a reduction in a temperature of the reaction liquid.

### <Removal of Impurities and Washing of Silica Gel>

In the present invention, after finishing the neutralization reaction, the reaction liquid of the W/O type emulsion including the alkali silicate aqueous solution and the mineral acid aqueous solution is heated as it is while being stirred without separating the generated spherical silica gel. Since this operation separates the reaction liquid in the emulsion state into the oil phase and the water phase (a mineral acid aqueous solution phase) including the spherical silica gel, the oil phase is removed from the above-mentioned reaction liquid and the mineral acid aqueous solution phase including the spherical silica gel is washed by pure water or the like, thereby allowing highly pure spherical silica gel to be obtained. In this case, it is required to heat the reaction liquid at a temperature of 50°C or more, and in consideration of processing time, it is preferable that preferably, the reaction liquid is retained at a temperature of 50°C to 120°C and more preferably, the reaction liquid is retained at a temperature of 80°C to 100°C for 30 minutes to one hour.

Note that in the description of the present application, a range of numerical values (rates) shown by using "to" shows a range including numerical values (rates) described before and after "to" as a minimum value (rate) and a maximum value (rate).

### <Drying of Silica Gel>

Moisture in the spherical silica gel from which the impurities have been removed by the above-described washing processing step is retained. The moisture retained in this silica gel is divided into adhesion water and structural water, and although the adhesion water can be easily removed by heating the silica gel at a temperature of approximately 100°C, it is difficult to completely remove the structural water by heating at even at a temperature higher than 400°C. However, if a drying temperature is made higher than 500°C or drying time is made longer than 40 hours, densification and clumping of the silica particles proceed, a specific surface area and a pore volume are thereby greatly decreased, and as a result, porous silica particles having particle shapes and hardness which are suited to obtain smooth texture to skin cannot be obtained.

Therefore, in the present invention, in a step after drying, without subjecting the silica particles to calcination processing at a temperature of 1000°C or more, by retaining the silica particles, preferably, at a temperature of 50°C to 500°C, and more preferably, at a temperature of 100°C to 400°C for, preferably, one minute to 40 hours, and more preferably, 10 hours to 30 hours, it is preferable that the spherical silica gel is subjected to only drying processing. As a result, as to the spherical silica of the present invention, the decrease in the specific surface area and the decrease in the pore volume, which are caused by the proceeding of densification and the like, are suppressed, and the spherical silica has a characteristic in that the content percentage of structural water is 1.6% or more while spherical shapes and hardness which are suited to obtain smooth texture to skin are achieved.

In addition, in order for the porous spherical silica particles obtained by drying to exhibit smooth texture to skin, it is preferable that preferably, the porous spherical silica particles have an average primary particle diameter of 0.1 µm to 20 µm and more preferably, an average primary particle diameter of 0.5 µm to 10 µm. If the average primary particle diameter of the silica particles becomes large, exceeding 20 µm, the particles applied or attached to skin easily fall by self-weight, thereby causing occurrence of cosmetic unevenness. On the other hand, if the average primary particle diameter becomes smaller than 0.1 µm, the particles enter depressed portions such as pores and wrinkles of skin, thereby leading to a problem in that it is made difficult to completely remove a cosmetic.

In order to make the spherical silica particles have the average primary particle diameter in the above-mentioned range, when drying is conducted by using, for example, a fluidized dryer, since the particles are crushed while being dried, there may be a case where in the step after drying, it is not needed to crush the particles. In addition, in a case where the particles are dried in a state in which the particles are left at rest, a case where the average primary particle diameter is controlled to be further precise, and other case, in the step after drying, the particles may be crushed or may be classified.

The spherical silica particles of the present invention have characteristics in that the specific surface area obtained by employing the BET method is 300 m²/g or more, the total pore volume is 0.3 ml/g or less, and the oil absorption is 50 ml/100g or less. In other words, the oil absorption is suppressed while the spherical silica particles of the present invention are porous and have the large specific surface area, and the particles are hardly broken even when used in cosmetic applications, thereby allowing the spherical silica particles to exhibit use feeling excellent in adherability to skin and texture. In addition, in order to obtain such spherical silica particles, conducting only the drying processing of the generated silica gel and omitting the calcination processing are effective.

A method for generating the silica gel particles which are subjected to the drying processing is not limited to the above-described method in which the alkali silicate is emulsified and coagulated, and a manufacturing method, other than the above-described method, such as a sol-gel method can also be employed. In the manufacturing method using the sol-gel method, as long as a raw material such as silicon alkoxide, alkali silicate, and silica sol is in a solution state or a sol state, by employing a method in which seed particles are grown, a method in which the raw material is suspended or emulsified and coagulated, and other method, the above-mentioned raw material is used and the drying processing is conducted, thereby allowing the silica gel particles to be obtained.

### ADVANTAGEOUS EFFECTS OF THE INVENTION

According to the present invention, porous spherical silica particles whose specific surface area obtained by employing a BET method is 300 m²/g or more, total pore volume is 0.3 ml/g or less, and oil absorption is 50 ml/100g or less can be obtained.

In addition, since the oil absorption thereof is suppressed while the spherical silica particles of the present invention are porous and have the large specific surface area, and particles are hardly broken even when used in cosmetic applications, use feeling excellent in adherability to skin and texture can be exhibited. Therefore, the spherical silica particles of the present invention can be suitably used as a texture improver of cosmetics.

### DESCRIPTION OF EMBODIMENT

Hereinafter, with reference to specific examples, spherical silica particles and a method for manufacturing spherical silica particles according to the present invention will be described in detail. Note that the present invention is not limited to an embodiment set forth below, and a variety of modifications can be made without departing from the technical ideas of the present invention.

### [EXAMPLES]

### (Example 1)

### <Step 1: Preparation of Emulsion>

100 grams of a non-polar solvent (xylene), 4 grams of an emulsifying agent (sorbitan monostearate), and 400 grams of a No. 1 silicate soda aqueous solution (having a concentration of 10 wt.% expressed in terms of SiO₂) were stirred by using an emulsifying apparatus (T.K. ROBOMIX manufactured by PRIMIX Corporation) at the number of revolutions of 4500 rpm for five minutes, thereby preparing emulsion.

### <Step 2: Generation of Silica Gel and Removal of Impurities>

500 grams of the emulsion obtained in step 1 were added while 500 grams of a sulfuric acid aqueous solution having a concentration of 40 wt.% was being stirred; the resultant was stirred for 30 minutes under a room temperature; and thereafter, the resultant was heated to 90°C under stirring and was retained for 30 minutes, thereby separating reaction liquid in an emulsion state to an oil phase and a water phase including a spherical silica gel. The oil phase was removed from the separated reaction liquid; the water phase including the silica gel was washed by pure water until electric conductivity in the water phase reached 80 µS/cm or less; and thereafter, dehydration was conducted, thereby obtaining the silica gel.

### <Step 3: Drying of Silica Gel>

The silica gel obtained in step 2 was dried at 120°C for 24 hours, thereby preparing spherical silica particles in Example 1.

### (Example 2)

Similar operations were conducted under the same conditions in Example 1 except that as a silicate soda aqueous solution, a No. 2 silicate soda aqueous solution was used, thereby preparing spherical silica particles in Example 2.

### (Example 3)

Similar operations were conducted under the same conditions in Example 1 except that as a silicate soda aqueous solution, a No. 3 silicate soda aqueous solution was used, thereby preparing spherical silica particles in Example 3.

### (Example 4)

Similar operations were conducted under the same conditions in Example 2 except that the heating temperature in step 2 was 70°C, thereby preparing spherical silica particles in Example 4.

### (Example 5)

Similar operations were conducted under the same conditions in Example 2 except that the heating temperature in step 2 was 50°C, thereby preparing spherical silica particles in Example 5.

### (Example 6)

Similar operations were conducted under the same conditions in Example 2 except that a concentration of the used silicate soda aqueous solution expressed in terms of SiO₂ was 5 wt.%, thereby preparing spherical silica particles in Example 6.

### (Example 7)

Similar operations were conducted under the same conditions in Example 2 except that a concentration of the used silicate soda aqueous solution expressed in terms of SiO₂ was 20 wt.%, thereby preparing spherical silica particles in Example 7.

### (Example 8)

Similar operations were conducted under the same conditions in Example 2 except that a concentration of the used sulfuric acid aqueous solution was 30 wt.%, thereby preparing spherical silica particles in Example 8.

### (Example 9)

Similar operations were conducted under the same conditions in Example 2 except that a concentration of the used sulfuric acid aqueous solution was 50 wt.%, thereby preparing spherical silica particles in Example 9.

### (Example 10)

Similar operations were conducted under the same conditions in Example 2 except that as an emulsifying agent, sorbitan monopalmitate was used, thereby preparing spherical silica particles in Example 10.

### (Example 11)

Similar operations were conducted under the same conditions in Example 2 except that as an emulsifying agent, sorbitan monolaurate was used, thereby preparing spherical silica particles in Example 11.

### (Example 12)

Similar operations were conducted under the same conditions in Example 2 except that as an emulsifying agent, sorbitan distearate was used, thereby preparing spherical silica particles in Example 12.

### (Example 13)

Similar operations were conducted under the same conditions in Example 2 except that as an emulsifying agent, sorbitan tristearate was used, thereby preparing spherical silica particles in Example 13.

### (Example 14)

Similar operations were conducted under the same conditions in Example 2 except that as an emulsifying agent, sorbitan monooleate was used, thereby preparing spherical silica particles in Example 14.

### (Example 15)

Similar operations were conducted under the same conditions in Example 14 except that an amount of a used emulsifying agent was 4.8 grams, thereby preparing spherical silica particles in Example 15.

### (Example 16)

Similar operations were conducted under the same conditions in Example 14 except that an amount of a used emulsifying agent was 3.2 grams, thereby preparing spherical silica particles in Example 16.

### (Example 17)

Similar operations were conducted under the same conditions in Example 2 except that as an emulsifying agent, sorbitan sesquioleate was used, thereby preparing spherical silica particles in Example 17.

### (Example 18)

Similar operations were conducted under the same conditions in Example 2 except that as an emulsifying agent, sorbitan trioleate was used, thereby preparing spherical silica particles in Example 18.

### (Example 19)

Similar operations were conducted under the same conditions in Example 2 except that the silica particles obtained in step 3 was further dried at a temperature of 350°C for three hours, thereby preparing spherical silica particles in Example 19.

### (Example 20)

Similar operations were conducted under the same conditions in Example 2 except that 0.20 gram of a titanium oxide (manufactured by TAYCA CORPORATION: with a brand of MT-150AW) was added to a silicate soda aqueous solution, the resultant was sufficiently mixed, and thereafter, an operation in step 1 was conducted, thereby preparing silica particles in Example 20.

### (Example 21)

Similar operations were conducted under the same conditions in Example 20 except that an added amount of a titanium oxide (manufactured by TAYCA CORPORATION: with a brand of MT-150AW) was 4.44 grams, thereby preparing silica particles in Example 21.

### (Example 22)

Similar operations were conducted under the same conditions in Example 20 except that an added amount of a titanium oxide (manufactured by TAYCA CORPORATION: with a brand of MT-150AW) was 17.14 grams, thereby preparing silica particles in Example 22.

### (Comparative Example 1)

Similar operations were conducted under the same conditions in Example 2 except that silica particles obtained in step 3 were subjected to calcination at a temperature of 1100°C for three hours, thereby preparing silica particles in Comparative Example 1.

### (Comparative Example 2)

Similar operations were conducted under the same conditions in Example 2 except that silica particles obtained in step 3 were subjected to calcination at a temperature of 650°C for three hours, thereby preparing silica particles in Comparative Example 2.

### (Comparative Example 3)

In order to compare the silica gel with one obtained by wet water-based synthesis, commercially available silica particles (manufactured by TOSOH SILICA CORPORATION: Nipsil E-743) were used as silica particles in Comparative Example 3.

### (Comparative Example 4)

In order to make a comparison with silica particles having an average primary particle diameter and a specific surface area which are equivalent to those in Example 1, commercially available silica particles (manufactured by AGC Si-Tech Co., Ltd.: H-52) were used as silica particles in Comparative Example 4.

### <Physical Property Evaluation Method >

### (Average Primary Particle Diameter)

Average primary particle diameters of silica particles in Examples and Comparative Examples were measured by using a scanning electron microscope.

Specifically, photographs of approximately 1000 silica particles, which were shot by using a scanning electron microscope (manufactured by Hitachi High-Tech Corporation: S-4800) were subjected to analysis by using image analysis-type particle size distribution software (manufactured by MOUNTECH Co., Ltd.: Mac-VIEW).

### (Specific Surface Area)

Specific surface areas of silica particles in Examples and Comparative Examples were measured by using a specific surface area measuring device (manufactured by MOUNTECH Co., Ltd.: Macsorb HM-model 1210).

Specifically, in order to remove moisture and the like physically adsorbed on surfaces and inside pores of silica particles, as pretreatment before the measurement, the silica particles were dried under a condition of temperature of 105°C for 12 hours and were left to be cooled in a desiccator. The cooled specimens were deaerated by nitrogen gas under a condition of a temperature of 150°C for 20 minutes, and specific surface areas were measured.

The specific surface areas were obtained by applying a calculation formula in a BET one-point method.

### (Total Pore Volume)

Total pore volumes of silica particles in Examples and Comparative Examples were measured by using a pore distribution measuring device (manufactured by MicrotracBEL Corp.: BELSORP mini).

Specifically, in order to remove moisture and the like physically adsorbed on surfaces and in pores of silica particles, first, the silica particles were subjected to vacuum drying under conditions of a degree of vacuum of 10⁻² kPa and a temperature of 150°C for 30 minutes.

Each of the total pore volumes was obtained by converting an adsorption amount of N₂ gas at a relative pressure of p/p₀=0.990 into a volume of N₂ in a liquid state.

### (Oil Absorption)

Each oil absorption of the silica particles in Examples and Comparative Examples was measured by employing a method described in JIS K 5101-13-2.

### (Content Percentage of Structural Water)

Content percentages of structural water of the silica particles in Examples and Comparative Examples were measured by using a thermogravimetric/differential thermal (TG-DTA) analyzer TG/DTA 6300 (manufactured by Seiko Instrument Inc.).

Specifically, in order to remove moisture and the like physically adsorbed on surfaces and in pores of silica particles, as pretreatment before the measurement, the silica particles were dried under a condition of temperature of 105°C for 12 hours and were left to be cooled in a desiccator. A difference between a weight reduction percentage (%) from a room temperature to a temperature of 500°C and a weight reduction percentage (%) from a room temperature to a temperature of 1100°C, measured when a temperature of the cooled silica particles was increased in the air (a flow rate: 200 ml/minute) at a temperature increasing speed of 10°C /minute from a room temperature to 1200°C, was defined as a content percentage of the structural water (%).

### (Texture Evaluation of Each of Powders upon Applying to Skin)

As to each of powders in Examples and Comparative Examples, adherability to skin and texture were evaluated by a sensory test by five monitors. Specifically, the adherability to skin and the texture sensed when a small amount of each of the powders was taken and applied to the back of the hand with his or her finger were evaluated with the following criteria and an average value was calculated.

### <Criteria of Evaluation Points>

Five points: extremely excellent
Four points: excellent
Three points: fair
Two points: inferior
One point: extremely inferior

Manufacturing conditions of each of the powders in Examples and Comparative Examples are shown in Table 1 and evaluation results thereof are shown in Table 2.

**[Table 1]**

| | Manufacturing conditions | | | | | | | | | | |
|---|---|---|---|---|---|---|---|---|---|---|---|
| | Preparation of emulsion | | | | | | | Synthesis of silica gel | | Drying | Calcination |
| | Oil phase | Kind of emulsifying agent | Added amount of emulsifying agent (to SiO₂) | Kinds of silicate soda | Concentration of silicate soda (expressed in terms of SiO₂) | Composite metal oxide | Content rate (to the whole particles) | Concentration of sulfuric acid | Heating temperature | Drying temperature | Calcination temperature |
| Example 1 | Xylene | Sorbitan monostearate | 10% | No. 1 | 10% | - | - | 40% | 90°C | 120°C | - |
| Example 2 | Xylene | Sorbitan monostearate | 10% | No. 2 | 10% | - | - | 40% | 90°C | 120°C | - |
| Example 3 | Xylene | Sorbitan monostearate | 10% | No. 3 | 10% | - | - | 40% | 90°C | 120°C | - |
| Example 4 | Xylene | Sorbitan monostearate | 10% | No. 2 | 10% | - | - | 40% | 70°C | 120°C | - |
| Example 5 | Xylene | Sorbitan monostearate | 10% | No. 2 | 10% | - | - | 40% | 50°C | 120°C | - |
| Example 6 | Xylene | Sorbitan monostearate | 10% | No. 2 | 5% | - | - | 40% | 90°C | 120°C | - |
| Example 7 | Xylene | Sorbitan monostearate | 10% | No. 2 | 20% | - | - | 40% | 90°C | 120°C | - |
| Example 8 | Xylene | Sorbitan monostearate | 10% | No. 2 | 10% | - | - | 30% | 90°C | 120°C | - |
| Example 9 | Xylene | Sorbitan monostearate | 10% | No. 2 | 10% | - | - | 50% | 90°C | 120°C | - |
| Example 10 | Xylene | Sorbitan monopalmitate | 10% | No. 2 | 10% | - | - | 40% | 90°C | 120°C | - |
| Example 11 | Xylene | Sorbitan monolaurate | 10% | No. 2 | 10% | - | - | 40% | 90°C | 120°C | - |
| Example 12 | Xylene | Sorbitan distearate | 10% | No. 2 | 10% | - | - | 40% | 90°C | 120°C | - |
| Example 13 | Xylene | Sorbitan tristearate | 10% | No. 2 | 10% | - | - | 40% | 90°C | 120°C | - |
| Example 14 | Xylene | Sorbitan monooleate | 10% | No. 2 | 10% | - | - | 40% | 90°C | 120°C | - |
| Example 15 | Xylene | Sorbitan monooleate | 12% | No. 2 | 10% | - | - | 40% | 90°C | 120°C | - |
| Example 16 | Xylene | Sorbitan monooleate | 8% | No. 2 | 10% | - | - | 40% | 90°C | 120°C | - |
| Example 17 | Xylene | Sorbitan sesquioleate | 10% | No. 2 | 10% | - | - | 40% | 90°C | 120°C | - |
| Example 18 | Xylene | Sorbitan trioleate | 10% | No. 2 | 10% | - | - | 40% | 90°C | 120°C | - |
| Example 19 | Xylene | Sorbitan monostearate | 10% | No. 2 | 10% | - | - | 40% | 90°C | 350°C | - |
| Example 20 | Xylene | Sorbitan monostearate | 10% | No. 2 | 10% | Titanium dioxide | 0.5% | 40% | 90°C | 120°C | - |
| Example 21 | Xylene | Sorbitan monostearate | 10% | No. 2 | 10% | Titanium dioxide | 10% | 40% | 90°C | 120°C | - |
| Example 22 | Xylene | Sorbitan monostearate | 10% | No. 2 | 10% | Titanium dioxide | 30% | 40% | 90°C | 120°C | - |
| Comparative Example 1 | Xylene | Sorbitan monostearate | 10% | No. 2 | 10% | - | - | 40% | 90°C | 120°C | 1100°C |
| Comparative Example 2 | Xylene | Sorbitan monostearate | 10% | No. 2 | 10% | - | - | 40% | 90°C | 120°C | 650°C |
| Comparative Example 3 | - | - | - | - | - | - | - | - | - | - | - |
| Comparative Example 4 | - | - | - | - | - | - | - | - | - | - | - |

**[Table 2]**

| | Evaluation | | | | | |
|---|---|---|---|---|---|---|
| | Specific surface area (m²/g) | Average primary particle diameter (µm) | Total pore volume (ml/g) | Oil absorption (ml/100g) | Content percentage of structural water (%) | Texture evaluation (5 points maximum) |
| Example 1 | 618 | 5.0 | 0.28 | 23.5 | 2.2 | 4.0 |
| Example 2 | 462 | 5.0 | 0.22 | 20.5 | 2.0 | 4.6 |
| Example 3 | 362 | 5.0 | 0.18 | 19.5 | 1.9 | 4.2 |
| Example 4 | 451 | 5.0 | 0.19 | 19.2 | 2.1 | 4.8 |
| Example 5 | 441 | 5.0 | 0.21 | 18.9 | 2.4 | 4.6 |
| Example 6 | 429 | 3.9 | 0.19 | 19.3 | 1.8 | 4.8 |
| Example 7 | 480 | 5.5 | 0.22 | 19.6 | 2.1 | 4.6 |
| Example 8 | 445 | 5.0 | 0.21 | 19.0 | 2.1 | 4.6 |
| Example 9 | 401 | 5.0 | 0.19 | 19.1 | 1.7 | 4.8 |
| Example 10 | 359 | 3.3 | 0.21 | 18.5 | 2.1 | 4.0 |
| Example 11 | 374 | 5.0 | 0.20 | 23.5 | 2.1 | 4.2 |
| Example 12 | 413 | 5.0 | 0.19 | 21.0 | 2.1 | 4.8 |
| Example 13 | 432 | 5.1 | 0.20 | 19.0 | 2.0 | 4.6 |
| Example 14 | 508 | 5.4 | 0.26 | 19.0 | 2.1 | 4.2 |
| Example 15 | 468 | 1.1 | 0.26 | 21.0 | 2.1 | 4.0 |
| Example 16 | 536 | 11.0 | 0.26 | 17.0 | 2.0 | 4.2 |
| Example 17 | 408 | 4.3 | 0.18 | 19.0 | 1.9 | 4.8 |
| Example 18 | 435 | 5.0 | 0.21 | 19.0 | 1.9 | 4.6 |
| Example 19 | 459 | 5.0 | 0.20 | 20.5 | 2.0 | 4.6 |
| Example 20 | 412 | 5.3 | 0.20 | 19.8 | 1.9 | 4.6 |
| Example 21 | 510 | 5.1 | 0.21 | 21.4 | 1.8 | 4.6 |
| Example 22 | 620 | 5.0 | 0.24 | 22.5 | 1.7 | 4.2 |
| Comparative Example 1 | 1 | 5.0 | 0.005 | 5.3 | 0.1 | 2.0 |
| Comparative Example 2 | 218 | 5.0 | 0.12 | 18.3 | 1.5 | 3.0 |
| Comparative Example 3 | 55 | 0.1 | 0.33 | 130.0 | 2.0 | 1.0 |
| Comparative Example 4 | 483 | 5.4 | 1.60 | 247.0 | 1.2 | 3.0 |

It was found from Tables 1 and 2 that as to the spherical silica particles in each of Examples 1 to 19, while in order to obtain excellent texture evaluation, the specific surface area is 300 m²/g or more (for example, in Examples 3 and 11) and more preferably, the specific surface area is 400 m²/g or more (for example, in Examples 4, 6, 9, 12, and 17), and the pore volume is 0.3 ml/g or less (for example, in Examples 14 and 16) more preferably, the pore volume is 0.25 ml/g or less (for example, in Examples 2, 7, 8, and 18) and further preferably, the pore volume is 0.2 ml/g or less (for example, in Examples 4, 6, 9, 12, and 17), and in the comparison with Comparative Examples 3 and 4 described later in particular, the oil absorption is suppressed to 50 ml/100g or less; more preferably, the oil absorption is suppressed to 30 ml/100g or less; and further preferably, the oil absorption is suppressed to 20 ml/100g or less.

It is considered that the specific surface area obtained by employing the BET method is made to be 300 m²/g or more; and more preferably, the specific area is 400 m²/g or more, thereby allowing the proportion of the area of the silica particles contacting skin to be sufficiently small and when the silica particles are applied to skin, hardness of the silica particles is hardly felt, thereby obtaining the favorable texture.

In addition, it is considered that the specific surface area obtained by employing the BET method is 300 m²/g or more; and more preferably, the specific surface area is 400 m²/g or more, and the total pore volume is 0.3 ml/g or less; more preferably, the total pore volume is 0.25 ml/g or less; and further preferably, the total pore volume is 0.2 ml/g or less (in other words, while the specific surface area is made large, the pore volume is made small), thereby allowing the oil absorption to be suppressed to a lower level such as 50 ml/100g or less; more preferably, 30 ml/100g or less; and further preferably, 20 ml/100g or less and adverse influence (clumping of the silica particles and dry feeling of skin) due to excessive absorption of an oil content is hardly caused, thereby allowing excellent use feeling to be obtained.

As a result, the oil absorption thereof is suppressed while the spherical silica particles in each of Examples 1 to 19 are porous and have the large specific surface area, and the spherical silica particles in each thereof can exhibit excellent adherability to skin and excellent texture.

In addition, it was found from Example 19 that even in the second drying, preferably, as drying conditions, drying is conducted at a temperature of 50°C to 500°C; more preferably, the drying is conducted at a temperature of 100°C to 400°C, and preferably, retainment is conducted for one minute to 40 hours; and more preferably, the retainment is conducted for 10 hours to 30 hours, and the oil absorption thereof is thereby suppressed while the spherical silica particles have the large specific surface area, thus allowing spherical silica particles of the present invention, which exhibit excellent adherability to skin and excellent texture, to be obtained.

Furthermore, it was found from Examples 20 to 22 that as to the silica particles obtained by compounding the titanium oxide, the oil absorption thereof is suppressed while the silica particles have the large specific surface area, thereby obtaining the spherical silica particles of the present invention, which exhibit excellent adherability to skin and excellent texture.

On the other hand, it is inferred that because the silica particles in Comparative Example 1 were subjected to the calcination at the temperature of 1100°C, densification of the particles and mutual sintering of the particles proceed, thereby greatly decreasing the specific surface area and the pore volume and further causing high hardening of the particles and mutual fusion of the particles. Therefore, it was found that the silica particles in Comparative Example 1 cannot exhibit excellent adherability to skin and excellent texture, thereby worsening use feeling.

It was found that although because the silica particles in Comparative Example 2 were subjected to the calcination at the comparatively low temperature of 650°C, a decrease in the specific surface area and a decrease in the pore volume caused by proceeding of densification of the particles and mutual sintering of the particles were suppressed to some extent, a large specific surface area cannot be obtained and as a result, excellent adherability to skin and excellent texture cannot be obtained.

In addition, it was found that although the specific surface area of the silica particles in Comparative Example 3 was 55 m²/g and the pore volume thereof was also 0.33 ml/g, showing the small values, in contrast thereto, because the oil absorption thereof was high, showing 130.0 ml/100g, the oil content of skin is, for example, excessively absorbed and as a result, mutual clumping of the silica particles and drying of a surface of skin were caused, whereby excellent adherability to skin and excellent texture cannot be obtained.

It was found that as with the silica particles in Comparative Example 1, although the silica particles in Comparative Example 4 have the specific surface area equivalent to that of the spherical silica particles in each of Examples 1 to 19, the pore volume thereof is 1.60 ml/g, showing the large value and in accordance therewith, the oil absorption thereof was 247.0 ml/100g, showing the high oil absorption. Therefore, also the silica particles in Comparative Example 4 excessively absorb the oil content of skin and as a result, mutual clumping of the silica particles and drying of a surface of skin were caused, thereby resulting in the adherability to skin and the texture inferior to the spherical silica particles in each of Examples 1 to 19.

As described above, it was found that in order to enhance use feeling such as rolling properties, adherability, and texture to skin upon compounding silica particles in general into a cosmetic or the like, the silica particles in general are porous, having the large specific surface area and the large oil absorption (Comparative Example 4) or while the silica particles in general have the small specific surface area and the small pore volume owing to pore control, the oil absorption thereof is large (Comparative Example 3), and spherical silica particles excellent in use feeling such as adherability and texture owing to suppression in oil absorption while the spherical silica particles have the large specific surface area as in each of Examples 1 to 19 have not so far been available as the silica particles in general.

In addition, it was found by the comparison with Comparative Examples 1 and 2 that in order to obtain the spherical silica particles having characteristics like those in each of Examples 1 to 19, subjecting silica gel particles obtained by employing a sol-gel method, for example, in which an alkali silicate is emulsified and coagulated, to only drying at a low temperature without subjecting the silica gel particles to calcination at a high temperature is effective.

## Claims

1. Spherical silica particles whose specific surface area being obtained by employing a BET method is 300 m²/g or more, total pore volume is 0.3 ml/g or less, and oil absorption is 50 ml/100g or less.

2. The spherical silica particles according to claim 1, wherein a content percentage of structural water is 1.6% or more.

3. The spherical silica particles according to claim 1 or 2, wherein the spherical silica particles are not subjected to calcination processing at a temperature of 1000°C or more.

4. The spherical silica particles according to any one of claims 1 to 3, wherein one kind or more of a metal oxide or metal oxides selected from the group consisting of a titanium oxide, a zinc oxide, an iron oxide, and an aluminum oxide is or are compounded.

5. The spherical silica particles according to claim 4, a content rate of the metal oxide or metal oxides to a whole of the silica particles is 0.5 wt.% to 30 wt.%.

6. A method for manufacturing spherical silica particles comprising:
(1) a step of forming a W/O type emulsion in which an alkali silicate aqueous solution is a dispersed phase and a liquid which does not mix with the alkali silicate aqueous solution is a continuous phase;
(2) a step of generating a spherical silica gel by mixing the W/O type emulsion with a mineral acid aqueous solution;
(3) a step of performing separation into two layers which are an O phase and a W phase and removing the O phase by heating a reaction liquid including the generated spherical silica gel;
(4) a step of washing the W phase including the spherical silica gel from which the O phase has been removed; and
(5) a step of drying the washed spherical silica gel.

7. The method for manufacturing spherical silica particles according to claim 4, further comprising a step of crushing the dried spherical silica particles.

8. The method for manufacturing spherical silica particles according to claim 4 or 5, wherein no calcination step is included after the (5) step.

9. The method for manufacturing spherical silica particles according to any one of claims 6 to 8, wherein the liquid which does not mix with the alkali silicate aqueous solution is a non-polar solvent.

10. The method for manufacturing spherical silica particles according to any one of claims 6 to 9, further comprising, before the (1) step, a step of adding, to the alkali silicate aqueous solution, one kind or more of a metal oxide or metal oxides selected from the group consisting of a titanium oxide, a zinc oxide, an iron oxide, and an aluminum oxide or a precursor compound or precursor compounds of one kind or more of the metal oxide or metal oxides selected from the group consisting of the titanium oxide, the zinc oxide, the iron oxide, and the aluminum oxide.

11. A texture improver for cosmetics, comprising the spherical silica particles according to any one of claims 1 to 5.

12. A cosmetic in which the spherical silica particles according to any one of claims 1 to 5 are compounded.
